# EUROPÄISCHE PATENTANMELDUNG

(11) **EP 1 738 715 A1**
(43) Veröffentlichungstag der Anmeldung: **03.01.2007**
(21) Anmeldenummer: 06008005.8
(22) Anmeldetag: 18.04.2006
(51) Int. Cl.: A61C 13/00, G06F 19/00

(54) **Verfahren und System zum Begutachten einer Implantatsplanung**

(30) Priorität: 24.06.2005 DE 102005029454
(71) Anmelder: Implant & 3D Planungscenter GbR, 86444 Affing-Muehlhausen (DE)
(72) Erfinder: Kielhorn, Jan, Dr., 74629 Pfedelbach (DE); Liedtke, Marcel, 86447 Todtenweis (DE); Stachulla, Gerhard, 86444 Affing-Bergen (DE)
(74) Vertreter: Grünecker, Kinkeldey, Stockmair & Schwanhäusser Anwaltssozietät

(57) **Zusammenfassung**

Die Erfindung betrifft ein Verfahren zum Begutachten einer Implantatsplanung bei dem an einem Ort eine Implantatsplanung auf einem Computer erstellt wird und Daten mit Bezug zu der Implantatsplanung an einen zweiten Ort per Datenfernübertragung übertragen werden, so dass die Implantatsplanung an dem zweiten Ort visuell dargestellt werden kann. Weiterhin betrifft die Erfindung ein System zur Begutachtung von einer Implantatsplanung mit einem Computer an einem ersten Ort, auf dem eine oder mehrere Softwareprogramme installiert sind, mit denen eine Implantatsplanung erstellt werden kann, Datenfernübertragungsmitteln, mit denen Daten mit Bezug zu der Implantatsplanung an einen zweiten Ort per Datenfernübertragung übertragen werden können und mit Visualisierungsmitteln, wie etwa einem Computer oder einem Bildschirm, mit denen die Implantatsplanung an dem zweiten Ort visuell dargestellt werden kann.

## Beschreibung

Die Erfindung betrifft ein Verfahren und System zum Begutachten einer Implantatsplanung.

Zur Herstellung von Zahnimplantaten ist es möglich, eine Implantatsplanung auf einem Computer zu erstellen. Hierbei können beispielsweise computertomographische (CT-) Daten des Kiefers des Patienten herangezogen werden, um digitalisierte Daten, die den Knochen des Kiefers beschreiben, zur Verfügung zu haben.

Bei der Erstellung von den CT-Daten ist es bekannt, Schablonen im oder bei dem Kiefer des Patienten anzuordnen, mit denen Referenzorte und -größen zur Verfügung gestellt werden.

Ist die Form des Knochens anhand der Digitaldaten bekannt, kann softwareunterstützt eine Implantatsplanung durchgeführt werden. Hierbei wird Größe, Form, Position, Orientierung etc. eines Implantats in Bezug auf den Knochen simuliert bzw. festgelegt.

Dies wird üblicherweise von Zahnärzten durchgeführt, kann jedoch auch durch Zahntechniker erfolgen.

Mit Hilfe einer solchen Implantatsplanung kann dann sowohl das Implantat als auch eine Bohrschablone hergestellt werden, die dem behandelnden Zahnarzt bei der Erstellung der Bohrung für das Implantat eine Hilfestellung liefert, indem sie sowohl die Position der Bohrung als auch die Richtung der Bohrung anzugeben vermag.

Bevor ein Implantat und eine Bohrschablone hergestellt werden, ist es üblich bzw. sinnvoll oder notwendig, dass der behandelnde Zahnarzt die Implantatsplanung überprüft und/oder genehmigt, insbesondere wenn er sie nicht selber erstellt hat.

Aufgabe der vorliegenden Erfindung ist es, ein Verfahren und ein System zur Verfügung zu stellen, mit dem die Begutachtung von Implantatsplanungen möglichst einfach und schnell möglich ist. Diese Aufgabe wird gelöst durch ein Verfahren nach Anspruch 1 und ein System nach Anspruch 10.

Vorteilhafte Ausführungsformen sind in den abhängigen Ansprüchen offenbart.

Bei dem Verfahren werden Daten, die einen Bezug zu der Implantatsplanung aufweisen, von dem ersten Ort an den zweiten Ort mit Datenfernübertragungsmitteln übertragen. Dies kann beispielsweise die Übertragung von einem Dentallabor zu einer Zahnarztpraxis sein. Die Übertragung der Daten erlaubt die Darstellung der Implantatsplanung an dem zweiten Ort in visueller Form. Dadurch ist es für den Zahnarzt mit möglichst wenig Aufwand möglich, sich ein möglichst umfassendes Bild von der Implantatsplanung zu verschaffen und diese somit begutachten zu können.

In einer vorteilhaften Ausführungsform werden von dem Computer Daten erstellt, die Ansichten der Implantatsplanung sind und an den zweiten Ort übertragen, sodass diese unmittelbar dargestellt werden können. Dies können beispielsweise Bitmaps, MPEG-Dateien, JPEG-Dateien oder sonstige Ansichten oder Ansichtssequenzen (Filme) sein.

Alternativ oder zusätzlich ist es in einer bevorzugten Ausführungsform auch möglich, dass Daten, aus denen Ansichtsdaten erstellt werden können, von dem ersten an den zweiten Ort übermittelt werden. Dies können beispielsweise Daten sein, die die dreidimensionale Konfiguration des Knochens und des Implantats wiedergeben. Aus diesen Daten können mit entsprechenden Renderingverfahren Ansichtsdaten, d. h. Bilder oder Filmsequenzen am zweiten Ort erstellt werden, sodass diese angesehen werden können.

In einer bevorzugten Ausführungsform können auch Daten von dem zweiten Ort an den ersten Ort übertragen werden. Dies bedeutet, dass beispielsweise von der Zahnarztpraxis auch Daten in das Dentallabor übertragen werden können. Diese Daten können beispielsweise Instruktionen für Computer am ersten Ort enthalten, die den Computer beispielsweise veranlassen, neue Ansichten oder Ansichtssequenzen der Implantatsplanung zu erstellen und diese an den zweiten Ort zu übermitteln. Es können auch Instruktionsdaten sein, die den Computer am ersten Ort veranlassen, die
selben Ansichtsdaten zu erstellen, die am zweiten Ort erstellt werden. Es können jedoch auch Ansichtsdaten sein, die am zweiten Ort erstellt wurden und am ersten Ort dargestellt werden sollen.

Die von dem zweiten an den ersten Ort übermittelten Daten können auch Genehmigungsdaten sein, die eine Genehmigung der Implantatsplanung zum Ausdruck bringen sollen. Die Genehmigungsdaten umfassen beispielsweise Daten, die zur Identifizierung einer Person dienen. Dies kann beispielsweise eine PIN (persönliche Identifikationsnummer) oder eine digitale Signatur sein.

Zur Datenfemübertragung sind sämtliche Datenfernübertragungsmittel einsetzbar, von denen hier nur beispielhaft das Internet, Telefonleitungen, Mobilfunk, ISDN oder DSL genannt sollen.

In einer weiteren bevorzugten Ausführungsform ist es möglich, dass Audiosignale von dem ersten an den zweiten und/oder von dem zweiten an den ersten Ort übertragen werden. Die Audiosignale können über die selbe Datenfernübertragung übertragen werden. Die Daten können jedoch auch über eine separate Datenfernübertragungsverbindung (z. B. Telefon, Mobilfunk) übertragen werden.

Vorteilhaft ist weiterhin eine Ausführungsform, bei der am zweiten Ort ebenfalls ein (zweiter) Computer zur visuellen Darstellung der Implantatsplanung eingesetzt wird. Es sind jedoch auch Fernsehbildschirme einsetzbar.

Das System zur Begutachtung von einer Implantatsplanung umfasst einen Computer an einem ersten Ort, auf dem eine oder mehrere Softwareprogramme installiert sind, mit denen eine Implantatsplanung erstellt werden kann. Weiterhin sind Datenfemübertragungsmittel vorgesehen, mit denen Daten mit Bezug auf die Implantatsplanung an einen zweiten Ort per Datenfemübertragung übertragen werden können. Der erste Ort ist beispielsweise ein Dentallabor und der zweite Ort eine Zahnarztpraxis.

An dem zweiten Ort sind Visualisierungsmittel, wie etwa ein Computer oder ein Bildschirm oder Ähnliches vorgesehen, mit dem die Implantatsplanung an dem zweiten Ort visuell dargestellt werden kann.

Mit dem System ist es also mit möglichst geringem Aufwand möglich, dass ein Zahnarzt eine in einem Dentallabor erstellte Implantatsplanung begutachtet.

In einer vorteilhaften Ausführungsform des Systems sind an dem zweiten Ort Eingabemittel zum Eingeben von Daten vorgesehen, die entweder am zweiten Ort zur Erstellung von verschiedenen Visualisierungen der Implantatsplanung verwendet werden können oder die an den ersten Ort als Instruktionsdaten für den Computer als Genehmigungsdaten übertragen werden können.

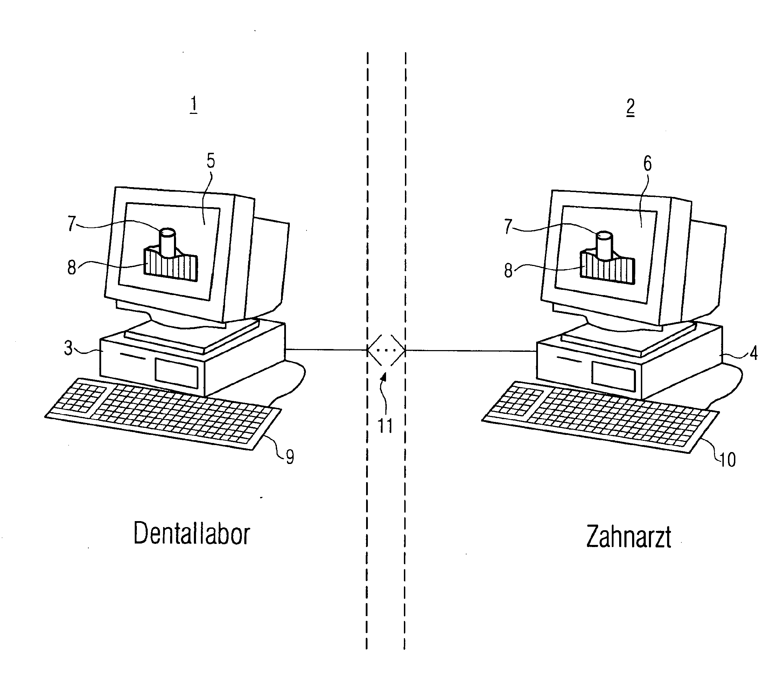

Vorteilhafte Ausführungsformen des Verfahrens und des Systems sollen anhand der beiliegenden Figur erläutert werden. Dabei zeigt die Figur eine schematische Darstellung des Systems.

Stellvertretend für den allgemeinen Fall soll hier konkret der Fall besprochen werden, bei dem der erste Ort der eines Dentallabors und der zweite Ort der einer Zahnarztpraxis ist.

Im Dentallabor 1 befindet sich ein Computer 3, auf dem eine Software zur Herstellung von Implantatsplanungen installiert ist. Es können auch verschiedene Softwareprogramme auf einem Rechner installiert sein, die zur Erstellung von Implantatsplanungen eingesetzt werden können und die wahlweise verwendet werden können.

In der Figur sind auf dem Bildschirm 5 des Computers 3 im Dentallabor 1 ein Knochenbereich 8 und ein Implantat 7 schematisch dargestellt. Die Implantatsplanung betrifft den Ort, die Orientierung (Winkel), Größe und Art des Implantats 7 relativ zu dem Knochenbereich 8. Der Knochenbereich 8 wird durch einen Datensatz beschrieben, der dreidimensional die Form des Knochens 8 wiedergibt. In der Figur ist auf dem Bildschirm 5 eine visuelle Ansicht der Implantatsplanung dargestellt. Hier ist das Implantat 7 in Relation zu dem Knochen 8 dargestellt.

Zusätzlich zu der visuellen Darstellung können für die Implantatsplanungen auch andere Daten, wie etwa Material des Implantats, Alter des Patienten, Knochenanomalitäten oder Ähnliches relevant sein, die beispielsweise in speziellen Datenfenstem oder Feldem auf dem Bildschirm 5 angezeigt werden können. Der Computer 3 ist über Datenfernübertragungsmittel 11 mit dem Computer 4 in der Zahnarztpraxis 2 verbunden. Dadurch ist es möglich, Daten, die mit dem Computer 3 erstellt wurden, an den Computer 4 zu übersenden, sodass in der Zahnarztpraxis 2 visuelle Ansichten der Implantatsplanung möglich sind. Hier können auch Daten wie Material des Implantats, Alter des Patienten, Knochenanomalien oder Ähnliches dargestellt werden.

In der Zahnarztpraxis 2 sind Eingabemitteln 10, wie etwa eine Tastatur, eine Mouse oder Ähnliches vorgesehen, mit denen Daten in den Computer 4 eingegeben werden können. Diese Daten können beispielsweise dazu verwendet werden, Ansichten auf die Implantatsplanung aus verschiedenen Richtungen zu erstellen, wobei die Ansichten von dem Computer 4 in der Zahnarztpraxis 2 oder von dem Computer 3 in dem Dentallabor 1 erstellt werden können.

Die Computer 3 und 4 können so miteinander gekoppelt werden, dass auf beiden Computern immer das selbe Bild dargestellt wird. Falls dann beispielsweise der Zahnarzt in der Zahnarztpraxis und ein Zahntechniker in dem Dentallabor miteinander telefonieren oder in anderer Art und Weise Audiodaten zwischen einander übermitteln, können sie so über die selben Ansichten sprechen.

Bei dem Verfahren wird im Dentallabor 1 eine Implantatsplanung erstellt. Daten mit Bezug zu der Implantatsplanung werden über Datenfernübertragungsmittel 11 an einen Computer 4 in einer Zahnarztpraxis 2 übertragen und dort auf einem Bildschirm 6 dargestellt. Die übertragenen Daten können beispielsweise ein 3D-Modell des Knochens 8 und des Implantats 7 sein, können aber auch lediglich Ansichten in Form von Bildern (zweidimensional) oder Bildsequenzen (Filme) sein. Werden dreidimensionale Modelldaten übertragen, können die Ansichten im Computer 4 erstellt werden und auf dem Bildschirm 6 ausgegeben werden.

Dann können in den Computer 4 mit Dateneingabemitteln 10 Daten eingegeben werden, die so oder in aufbereiteter Form an den Computer 3 im Dentallabor 1 übertragen werden. Dies kann beispielsweise dazu dienen, die Ansichten auf den beiden Computern 3, 4 zu synchronisieren oder den Computer 3 zu veranlassen, eine oder mehrere neue Ansichten der lmplantatsplanung zu erstellen.

Die Daten können auch Genehmigungsdaten sein, mit denen die Implantatsplanung von dem Zahnarzt genehmigt wird.

Die visuellen Darstellungen im Dentallabor 1 oder in der Zahnarztpraxis 2 können auch statt mit 2-dimensionalen Bildsschirmen mit Vorrichtungen zur Visualisierung von 3D- Daten erfolgen.

## Patentansprüche

1. Verfahren zum Begutachten einer Implantatsplanung bei dem
an einem ersten Ort (1) eine Implantatsplanung auf einem Computer (3) erstellt wird
und Daten mit Bezug zu der Implantatsplanung an einen zweiten Ort (2) per Datenfemübertragung (11) übertragen werden, so dass die Implantatsplanung an dem zweiten Ort (2) visuell dargestellt werden kann.

2. Verfahren nach Anspruch 1, **dadurch gekennzeichnet, dass** Ansichtsdaten, die eine Ansicht der Implantatsplanung darstellen, von dem ersten (1) an den zweiten Ort (2) übertragen werden.

3. Verfahren nach Anspruch 1 oder 2, **dadurch gekennzeichnet, dass** Daten, aus denen Ansichtsdaten erstellt werden können, von dem ersten (1) an den zweiten Ort (2) übertragen werden.

4. Verfahren nach einem der Ansprüche 1 bis 3, **dadurch gekennzeichnet, dass** von dem zweiten Ort (2) Daten an den ersten Ort (1) übermittelt werden, wie beispielsweise Instruktionsdaten, die Instruktionen für den Computer (3) beinhalten.

5. Verfahren nach Anspruch 4, **dadurch gekennzeichnet, dass** die Daten Genehmigungsdaten sind, die eine Genehmigung der Implantatsplanung zum Ausdruck bringen.

6. Verfahren nach Anspruch 5, **dadurch gekennzeichnet, dass** die Genehmigungsdaten, Daten zur eindeutigen Identifizierung der genehmigenden Person umfassen, wie etwa eine PIN oder eine digitale Signatur.

7. Verfahren nach einem der Ansprüche 1 bis 6, **dadurch gekennzeichnet, dass** Audiosignale von dem ersten Ort (1) an den zweiten Ort (2) und/oder von dem zweiten Ort (2) an den ersten Ort (1) übertragen werden.

8. Verfahren nach einem der Ansprüche 1 bis 7, **dadurch gekennzeichnet, dass** die Datenfernübertragung per Intemet, Telefonleitung, Mobilfunk, ISDN, DSL oder anderer Datenfernübertragungsmittel stattfindet.

9. Verfahren nach einem der Ansprüche 1 bis 8, **dadurch gekennzeichnet, dass** an dem zweiten Ort (2) ein zweiter Computer (4) zur visuellen Darstellung der Implantatsplanung eingesetzt wird.

10. System zur Begutachtung von einer Implantatsplanung mit:
einem Computer (3) an einem ersten Ort (1), auf dem eine oder mehrere Softwareprogramme installiert sind, mit denen eine Implantatsplanung erstellt werden kann,
Datenfemübertragungsmitteln (11), mit denen Daten mit Bezug zu der Implantatsplanung an einen zweiten Ort (2) per Datenfernübertragung übertragen werden können und mit
Visualisierungsmitteln, wie etwa einem Computer (4) oder einem Bildschirm, mit denen die Implantatsplanung an dem zweiten Ort (2) visuell dargestellt werden kann.

11. System nach Anspruch 10, **dadurch gekennzeichnet, dass** an dem zweiten Ort (2) Eingabemittel (10) zum Eingeben von Daten, wie etwa Instruktionsdaten für den Computer oder Genehmigungsdaten, vorgesehen sind.
